# EUROPEAN PATENT SPECIFICATION

(11) **EP 0 726 727 B1**
(45) Date of publication and mention of the grant of the patent: **21.04.2004**
(21) Application number: 94930855.5
(22) Date of filing: 13.10.1994
(51) Int. Cl.: A61B 1/303, A61B 1/313, A61B 17/32, A61B 17/425, A61B 17/42

(54) **TRANSVAGINAL TUBE AS AN AID TO LAPAROSCOPIC SURGERY**
TRANSVAGINAL-TUBUS ALS HILFSGERÄT FÜR LAPAROSKOPISCHE EINGRIFFE
TUBE TRANSVAGINAL FACILITANT UNE CHIRURGIE AU LAPAROSCOPE

(30) Priority: 22.10.1993 GB 9321773; 31.05.1994 AU PM601594
(43) Date of publication of application: 21.08.1996
(73) Proprietor: Gynetech Pty Ltd, West Perth, Western Australia (AU)
(72) Inventor: McCartney, Anthony John, Swanbourne, W.A. 6010 (AU)
(74) Representative: West, Alan Harry
(86) International application number: PCT/AU1994/000630
(87) International publication number: WO 1995/010973

(56) References cited:
- EP-A- 0 407 057
- WO-A-86/06968
- WO-A-93/24063
- AU-A- 378 621
- FR-A- 2 697 989
- US-A- 3 075 516
- US-A- 3 542 031
- US-A- 4 112 932
- US-A- 4 997 419
- US-A- 5 108 408
- DERWENT ABSTRACT, Accession No. 93-180703/22, class P31; & SU,A,1 739 977 (ROST ONCOLOGY INST), 15 June 1992.

## Description

THIS INVENTION relates to a transvaginal tube which is particularly useful in laparoscopic surgery, and also to a procedure for the use of such a tube.

Modern advances in laparoscopic surgical equipments have meant that surgeons are able to remove the uterus and/or ovaries laparoscopically, removing the need for a long abdominal incision.

A laparoscopic radical hysterectomy for cancer has evolved from the efforts of a few oncology centres with an interest in minimising invasive surgery. The operative technique is analogous to a modification of the operation originally described by Wertheim and Meigs. The laparoscope surgeon passes a 10mm laparoscope trans-abdominally through a sub-umbilical incision after establishing a pneumoperitineum. Using two lateral portals the ovarian pedicles are divided down to the level of the uterine arteries. The ureter is isolated and protected and the uterine vessels and parametrium are divided after mobilising the bladder. The next stage is to remove the uterus and close the vaginal vault so the pneumoperitoneum can be re-established for the lymphadenectomy. The lymph nodes are removed by plucking them from their bed and dragging them out of the abdomen through the trans-abdominal wall port used for the grasping forceps.

Various medical commentators suggest that laparoscopically assisted radical hysterectomy (colloquially known as "keyhole Wertheims") offers many advantages. Patients go home earlier and the convalescence period is shorter. The disadvantage is that the additional laparoscopic surgery increases operative time.

Plastic bags have been used to harvest ovaries in an attempt to minimise the contamination of metastatic material through the ports. However, these are fiddly to use and can be difficult to drag out of the abdominal port. Nonetheless, one study has demonstrated that ordinary plastic bags are just as effective as commercially available customised bags and ovaries and omentum can be placed in a bag and delivered through the vagina. This is an excellent refinement for ovarian surgery but it is difficult to place multiple small nodes in several bags and be sure of their origin.

EP 407057 describes a probe for applying electromagnetic radiation to the pelvic cavity. To allow this apparatus to be inserted into a vagina, a protective speculum is provided to prevent the metal end section of the probe contacting the vaginal wall. This speculum is open at both ends and has an angled rim at its inner end.

The present invention provides a transvaginal tube which is suitable for use in laparoscopic surgical techniques.

According to the invention, there is provided a transvaginal tube adapted for insertion into the vaginal tract of a patient for use during performance of a laparoscopic hysterectomy or other laparoscopic surgery on the patient, the tube having a distal end and a proximal end, the proximal end being cut in a plane non-normal to its tubular axis and being adapted to circumscribe the patient's cervico-vaginal junction, the tube further comprising a sealing means capable of forming a seal at the distal end of the tube during surgery, the tube being capable of maintaining the pneumoperitoneum when inserted into the vaginal tract of a patient with the seal formed at the distal end of the tube.

Preferably, the proximal end of the transvaginal tube is bevelled so that the leading or anterior edge of the tube protrudes beyond the posterior edge. In this respect, the anterior edge is, for example, 1 to 2 cm longer than the posterior edge. To avoid tissue damage in use, the edge of the tube surrounding the open bevelled end may be adapted to have a smooth edge. This may, for example, be achieved by moulding or shaping the tube with smooth convex edges or by attaching a cover means to the proximal end of the tube walls which blankets and provides a smooth surface over the wall of the bevelled open end of the tube.
The transvaginal tube may be formed of any material. However, the tube is preferably made of a plastic material which provides a degree of flexibility.

Preferably, the tube is also substantially transparent. It will be appreciated that the tube may also be formed of opaque material but may contain one or more transparent portals along the length of the tube.

The tube may be of any length and diameter. Preferably the tube is of a greater length than 5 cm and has a diameter of from 10 to 100 mm. More preferably, the tube is 25 to 50 cm in length and has a diameter of from 30 to 50 mm.

To facilitate use in a sterile environment, the transvaginal tube is preferably capable of withstanding sterilization. Any means known in the art which is capable of sealing the distal end of the tube may be employed in the invention. For example, the distal end may be capped or plugged. Preferably the sealing means is capable of effecting a fluid tight closure of the distal end of the tube to approximately 5 to 30 cm of water pressure but most preferably 15 cm of water pressure.

Alternatively, the distal end of the tube may be in a releasable sealing engagement with at least a valve means which when closed is capable of forming a fluid tight enclosure at the distal end of the tube to approximately 5 to 30 cm of water pressure, and most preferably to 15 cm of water pressure. Any valve means known in the art which is capable of achieving this may be employed in the invention. To facilitate use in a sterile environment, the valve means is preferably capable of withstanding sterilization.

When the distal end of the transvaginal tube is open, one or more smaller bore tubes may be inserted into the transvaginal tube. For example, a smaller bore tube may be fitted into the end of the transvaginal tube to promote washing of body fluids and tissue specimens from the proximal end of the interior of the transvaginal tube into a plastic collection bowl with or without the aid of suction when the transvaginal tube is fitted within a subject. Alternatively there may be passed into the transvaginal tube an intra-uterine manipulator which is longer than the internal length of the tube and which may be fixed by aids to the inside of the tube. The distal end of this manipulator may be used to enter the cervix to allow manipulation of the cervix throughout the surgical procedure of hysterectomy or adnexal surgery.

In another embodiment of the invention there is fixed in a concentric arrangement within the transvaginal tube a fluid tight channel through which a telescope or light sources may be inserted while maintaining fluid pressure within the tube. Preferably that channel extends from the distal end to the proximal end of the tube. The channel may be sealed at the distal end of the tube. Alternatively, the distal end of the tube may be adapted to house a portal which is capable of forming a releasable sealing engagement with the circumferential rim of the distal open end of the channel providing the interior of the channel with a means of communication with the exterior of the tube. The proximal end of the tube is sealed.

Desirably the releasable sealing engagement between the tube and the channel should be capable of withstanding approximately 5 to 30cm of water pressure but most preferably 15cm of water pressure. While such a channel may be suitable for telescopes and light sources it will be appreciated that other surgical instruments may be inserted in the channel.

In a further embodiment, when the interior of the channel is in communication with the exterior of the tube there is preferably provided at least a valve means inserted in the longitudinal wall of the tube. The valve means should be capable of withstanding 5 to 30 cm of water pressure but most preferably 15 cm of water pressure.

In yet a further embodiment of the invention there is releasably engaged to the longitudinal wall of the tube at least a valve means and there is passed in concentric arrangement through the distal end or longitudinal wall of the transvaginal tube and extending the length of the tube, smaller bore tubes which may, for example, provide passage for intra-uterine manipulators, tubes to promote irrigation of tissue, laparoscopic grasping forceps, laparoscopically directed stapling devices or laparoscopically directed electro-coagulation diatherny, laser or ultrasonic scalpel devices. To facilitate use in a sterile environment the tube is preferably sealed at the distal end and is preferably capable of withstanding sterilization. Any means known in the art for sealing the valve means and the smaller bore tubes may be employed with the invention. Preferably the tube comprises at least a valve means and one or more narrow diameter tubes, and when sealed at its distal end, is capable of withstanding approximately 5 to 30 cm of water pressure but most preferably 15cm of water pressure.

The present invention may be employed in laparoscopic surgery for exposure of the vaginal fornices, as an aid to separation of the bladder from the vagina, for division of the vagina by electro-coagulation diathermy, laser or ultrasonic scalpel, and as a conduit for exteriorising tissue from the abdominal pelvic cavity (see Tables 1 and 2). In this respect, the tissue may be the uterus and/or its adnexal, ovarian cysts, and particular pelvic lymph nodes. The present invention may also be employed as an exit for fluid such as blood and irrigation fluid and smoke or vapour from the abdominal pelvic cavity, as a splinting device to present and expose the dividing edges of the vagina, to facilitate suture closure as a splinting device in the vagina, to expose the vaginal fornices and lateral edge at bladder neck surgery, for insertion of sling devices and as a portal for telescopes or light tubes into the vagina while keeping them separate from the vaginal wall and exposing the vaginal mucoso through the substantially transparent walls of or transparent portals in the tube.

The tube may be used to outline the vagina at open surgery. In these cases the valved end (distal) is not required.

The transvaginal tube according to the invention finds use in a method for laparoscopic hysterectomy treatment comprising the steps of :
(i) introducing the transvaginal tube into the vagina of a patient so that the proximal end of the tube circumscribes the cervico-vaginal junction;
(ii) separating the cervix from the vagina; and
(iii) mobilizing a surgical specimen and inserting it into the proximal end of the tube.

Preferably the cervix is separated from the vagina by the steps of:
(i) exposing and holding the cervix with laparoscopic grasping manipulators;
(ii) inserting a knife or diathermy knife to the proximal end of the tube;
(iii) rotating the tube so the plane non-normal to the tubular axis stretches the vagina and at the same time cutting the cervico-vaginal junction as the tube is rotated.

As well as being most commonly used for simple laparoscopic hysterectomy, the transvaginal tube of the invention may also be used in laparoscopic radical hysterectomy and pelvic lymph node dissection.

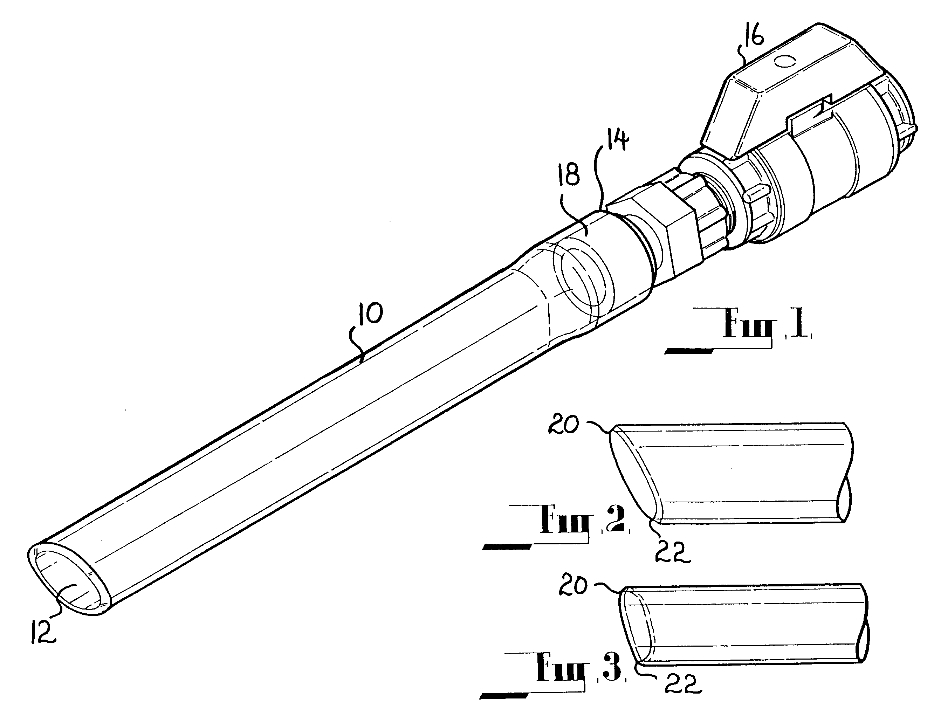

The transvaginal tube of the invention will now be described in relation to various examples, which in turn refer to the embodiment to the transvaginal tube as illustrated in the Figures 1 to 3. However, it must be appreciated that the following description is not to limit the generality of the above description.

The invention will be more fully understood in the light of the following description of one specific embodiment. The description is made with reference to the accompanying drawings of which:-
Figure 1 is an isometric view of a transvaginal tube;
Figures 2 and 3 are sectional view of the proximal end of the transvaginal tube; and
Figure 4 is a sectional view of the proximal end of the transvaginal tube in situ.

The embodiment (Figure 1) relates to a transvaginal tube which has a body defined by a tube 10 which has a proximal end 12 and a distal end 14. The distal end 14 engages a valve means 16 at a junction 18. The tube 10 is a relatively stiff yet flexible plastic material such as polypropylene. The junction 18 is provided by the resilience of the plastic material allowing for a force fit engagement of the distal end 14 of the tube 10 with one end of the valve means 16 so as to provide a substantially fluid tight engagement.

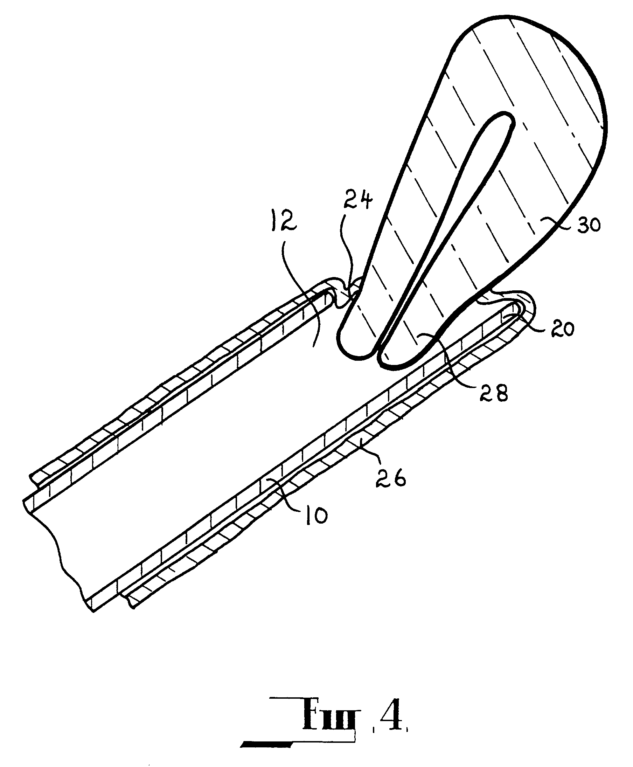

Figures 2 and 3 show sectional side views of the proximal end 12 of the tube 10 including an anterior edge 20 and a posterior edge 22. It will thus be apparent that the proximal end 12 is cut at an angle non-normal to the longitudinal axis of the tube. This provides the bevelled appearance that is apparent in Figures 2 and 3 and results in the anterior edge 20 being 1.5cm beyond the posterior edge 22.

Figure 4 shows the proximal end 12 of the tube 10 located insitu at the cervico-vaginal junction 24. When located in this position, the vaginal walls 26 envelope the tube 10 forming a seal around the tube. The cervix 28 protrudes into the proximal end 12 of the tube 10 and the uterus 30 lies above and exterior to the anterior edge 20. Thus it will be apparent that the proximal end 12 is adapted to circumscribe the cervico-vaginal junction.

### Example 1

A tube made of firm or flexible smooth transparent plastic, with a valve mounted at one end is sterilised in preparation for gynaecological laparoscopic surgery. When the patient is anaesthetized, positioned, cleansed and draped according to local custom, the tube is passed through the female vagina. The proximal end of the tube circumscribes the cervix from the vagina. The tube is removed to allow the uterus and appendages to be exteriorised, following which the tube is replaced and the proximal end of the tube is intraperitoneal. The distal end is closed with a valve and the pneumoperitoneum is maintained. No other device is needed to maintain the pneumoperitoneum but the tube is secured in place. Any fixation is acceptable but it is usually held by an assistant surgeon or one end is rested on a table. The tube is positioned according to need.

The proximal end of the tube when located intra-abdominally lies distal above the exteriorised end to allow surgical specimens to pass down the tube by gravity. The proximal end is placed close to the vaginal edges pointing slightly to the appropriate pelvic side wall during a pelvic lymphadenectomy but is placed further through the vagina for a cholecystectomy. The surgical specimen is mobilised and placed in the mouth of the tube. It is exteriorised as it slides down the tube. In this respect tissue may be encouraged to pass down the tube by washing it with irrigation fluid.

Blood and irrigation fluid that has collected in the cul de sac may also be exteriorised through the tube. As an additional aid the valve engaged to the distal end of the tube may be opened, releasing the pneumoperitoneum and the high intra-abdominal pressure relative to the environment would further expel free intra-abdominal and intratube contents. At the completion of the laparoscopic surgery the tube would be removed and the vagina closed. tube and specimen are removed. If the uterus is too large to be totally inserted into the tube, just the cervix is inserted and the specimen is steered to the introitus where it can be grasped by a vulsellum and removed.

Another advantage of the tube is that the vault can be sutured laparoscopically without resorting to intracorporial knots. Replacing the tube in the vagina supports and exposes the vaginal edges. The edges can be sutured by placing a needle, preferably taper-cut with a monofilament absorbable suture, in the tube so the trailing end remains intravaginal. The needle is recovered from the tube and the first stitch passes through the inside of the vagina to the peritoneal surface. The vault is closed with a purse string or a linear closure from one end to the other and back again. When the suturing is complete the needle is returned to the transvaginal tube so that both ends of the suture are in the vagina. The tube is withdrawn leaving the needle and trailing end of the suture at the introitus so the operator can lean over and tie the ends intravaginally. Other intracorporeal or extracorporeal techniques of knot tying may be employed.

### Results

The tube was used to separate the vagina from the cervix in forty cases by five different surgeons, thirty-four of these were simple laparoscopic hysterectomies for benign diseases in women with limited vaginal access, three were hysterectomies plus pelvic lymphadenectomy for endometrial cancer and three were modified radial hysterectomies for early cervical cancer. In twenty cases the vault was closed laparoscopically. There were no intraoperative complications attributable to the tube.

### Example 3

Use of the transvaginal tube as an aid to bladder neck surgery.

At either open or laparoscopic surgery, designed to elevate the bladder neck for treatment of stress incontinence of urine in the female, by either the Birch, Cato-Murray or similar procedure, the transvaginal tube previously described can also be used without the valve at the distal end to enhance bladder neck surgery. The tube may be any length from 5cms to 35cms in length, made of the same clear, rigid or semi-rigid plastic as previously described.

At that time in the procedure when the para-vesical area is being prepared to expose the lateral vaginal fornices, the tube is inserted with a telescope, either a direct (0 degrees) or a forward oblique type for example, with a 30 degree angle.

The tube acts to expand the vaginal walls, particularly the vaginal fornices so that they are made more prominent and fixed so that definite identification from either the open procedure or the retro-peritoneal laparoscopic or trans-peritoneal laparoscopic approach, is enhanced. The view of the vaginal fornix on the left and right is further enhanced by trans-illumination from the vaginal aspect by the telescope light. This enables improved exposure and ability to see and reflect the bladder wall from the vaginal fornices from above because of the trans-illumination effect. It also allows enhanced identification of the para vaginal venous plexus so that the large veins can be more easily avoided by the needle and suture placed from above into the vaginal wall. Placing of the Birch or Cato-Murray type suture through the sub-mucosal layers of the vagina is made easier as the large veins of the para-vaginal wall are made more identifiable by trans-illumination. With the vaginal telescope within the tube, connected to a camera and a screen, the appropriate site selected for insertion of the suture can be seen through the wall of the tube merely by compressing the wall with the outer part of the needle before insertion of the suture. The tube allows a broader and more secure bite of the wall to be taken and puncture of the mucosal or epidermal layer by the needle can be immediately recognised on the vaginal telescope screen. This supersedes the current practise of an assistant placing a thimble covered finger in the right and left vaginal fornices while the operator places the suture in the area of the vagina over the assistant's finger. With the vaginal fornices secured, tension can then be placed on the securing sutures and an appreciation of symmetrical bladder neck elevation can be made on the screen. At present, other than by the assistant's palpation as an assessment of the elevating effect, the surgeon has no idea of the degree of elevation or the right or left symmetry that is being obtained by the procedure.

In that case, full thickness penetration by the non-absorbable suture can only be discerned by the operator or the assistant feeling contact with the metal needle with the metal thimble or noting bleeding on the assistant's glove which would indicate full thickness puncture. In that case, the needle has to be withdrawn and re-inserted into the sub-mucosal dermis of the vagina.

## Claims

1. A transvaginal tube (10) adapted for insertion into the vaginal tract of a patient for use during performance of a laparoscopic hysterectomy or other laparoscopic surgery on the patient utilising electro-coagulation diathermy, the tube being of substantially stiff but flexible plastics material and having a distal end (14) and a proximal end (12), the proximal end being cut in a plane (20,22) non-normal to its tubular axis whereby the proximal end is angled and dimensioned to circumscribe the patient's cervico-vaginal junction (24) to facilitate separation of the vagina by electro-coagulation diathermy, the tube further comprising a sealing means (16) capable of forming a seal at the distal end (14) of the tube during surgery, the tube being capable of maintaining the pneumoperitoneum when inserted into the vaginal tract of a patient with the seal formed at the distal end of the tube.

2. A tube according to claim 1, wherein said seal is selectively releasable.

3. A tube according to claim 1 or claim 2, wherein the proximal end (12) of the tube is bevelled.

4. A tube according to any one of claims 1 to 3, wherein the proximal end (12) has a smooth edge.

5. A tube according to any one of claims 1 to 4, wherein the tube (10) is transparent.

6. A tube according to any one of claims 1 to 4, wherein the tube (10) is opaque and has one or more transparent portals along its length.

7. A tube according to any one of claims 1 to 6, wherein the sealing means (16) comprises a plug or cap.

8. A tube according to claim 7, wherein the plug or cap is capable of forming a seal at the distal end of the tube to approximately 5 to 30 cm of water pressure.

9. A tube according to claim 7, wherein the plug or cap is capable of forming a seal at the distal end of the tube to approximately 15 cm of water pressure.

10. A tube according to any one of claims 1 to 6, wherein the sealing means (16) comprises a valve means.

11. A tube according to claim 10, wherein the valve means is in releasable engagement with the distal end of the tube.

12. A tube according to claim 10 or claim 11, wherein the valve means is capable of forming a seal at the distal end of the tube to approximately 5 to 30 cm of water pressure.

13. A tube according to claim 10 or claim 11, wherein the valve means is capable of forming a seal at the distal end of the tube to approximately 15 cm of water pressure.

14. A tube according to any one of claims 1 to 13, adapted to receive at least one bore tube of smaller diameter than the tube (10) when the distal end of the tube is open.

15. A tube according to any one of claims 1 to 14, adapted to receive at least a surgical manipulator when the distal end of the tube is open.

16. A tube according to claim 15, adapted to have the surgical manipulator fixed to the inside of the tube.

17. A tube according to any one of claims 1 to 16, further comprising a fluid tight channel supported in concentric arrangement within the tube (10), the channel being sealed at its proximal end and open at its distal end and wherein the circumferential rim of the distal end of the channel is in sealing engagement with a portal formed in the distal end (14) of the tube (10) thus providing a means of communication between the interior of the channel and the exterior of the tube.

18. A tube according to claim 17, wherein the engagement between the channel and the tube is capable of withstanding approximately 5 to 30 cm of water pressure.

19. A tube according to claim 17 or claim 18, wherein the channel is adapted to receive a telescope.

20. A tube according to any one of claims 17 to 19, wherein the channel is adapted to receive at least a light source.

21. A tube according to any one of claims 17 to 20, further comprising a second valve means releasably engaged to the longitudinal wall of the tube.

## Patentansprüche

1. Transvaginaltubus (10) beschaffen zum Einführen in die Scheide einer Patientin zur Verwendung bei der Durchführung einer laparoskopischen Hysterektomie oder eines anderen laparoskopischen Eingriffs an der Patientin unter Verwendung von Elektrokoagulationsdiathermie, wobei der Tubus aus im Wesentlichen steifem, aber flexiblem Kunststoffmaterial ist und ein distales Ende (14) und ein proximales Ende (12) aufweist, wobei das proximale Ende in einer nicht senkrecht zur Tubusachse verlaufenden Ebene (20, 22) geschnitten ist, wodurch das proximale Ende gewinkelt und so bemessen ist, dass es die zervikal-vaginale Verbindungsstelle (24) umschreibt, um das Abtrennen der Scheide mittels Elektrokoagulationsdiathermie zu erleichtern, wobei der Tubus weiterhin eine Verschlusseinrichtung (16) umfasst, die im Stande ist, am distalen Ende (14) des Tubus während des Eingriffs einen Verschluss auszubilden, wobei der Tubus, wenn er in die Scheide einer Patientin eingeführt und der Verschluss am distalen Ende des Tubus ausgebildet ist, dazu in der Lage ist, das Pneumoperitoneum zu erhalten.

2. Tubus nach Anspruch 1, wobei der Verschluss selektiv erneut verschließbar ist.

3. Tubus nach Anspruch 1 oder 2, wobei das proximale Ende (12) des Tubus abgeschrägt ist.

4. Tubus nach einem der Ansprüche 1 bis 3, wobei das proximale Ende (12) einen glatten Rand aufweist.

5. Tubus nach einem der Ansprüche 1 bis 4, wobei der Tubus (10) durchsichtig ist.

6. Tubus nach einem der Ansprüche 1 bis 4, wobei der Tubus (10) undurchsichtig ist und eine oder mehrere durchsichtige Pforten in Längsrichtung aufweist.

7. Tubus nach einem der Ansprüche 1 bis 6, wobei die Verschlusseinrichtung (16) einen Stopfen oder eine Verschlusskappe umfasst.

8. Tubus nach Anspruch 7, wobei der Stopfen oder die Verschlusskappe im Stande ist, am distalen Ende des Tubus eine Abdichtung gegen ungefähr 5 bis 30 cm Wassersäule auszubilden.

9. Tubus nach Anspruch 7, wobei der Stopfen oder die Verschlusskappe im Stande ist, am distalen Ende des Tubus eine Abdichtung gegen ungefähr 15 cm Wassersäule auszubilden.

10. Tubus nach einem der Ansprüche 1 bis 6, wobei die Verschlusseinrichtung (16) eine Ventileinrichtung umfasst.

11. Tubus nach Anspruch 10, wobei die Ventileinrichtung in lösbarem Eingriff mit dem distalen Ende des Tubus steht.

12. Tubus nach Anspruch 10 oder 11, wobei die Ventileinrichtung im Stande ist, am distalen Ende des Tubus eine Abdichtung gegen ungefähr 5 bis 30 cm Wassersäule auszubilden.

13. Tubus nach Anspruch 10 oder 11, wobei die Ventileinrichtung im Stande ist, am distalen Ende des Tubus eine Abdichtung gegen ungefähr 15 cm Wassersäule auszubilden.

14. Tubus nach einem der Ansprüche 1 bis 13, beschaffen zur Aufnahme wenigstens eines Durchlasstubus mit einem kleineren Durchmesser als der Tubus (10), wenn das distale Ende des Tubus offen ist.

15. Tubus nach einem der Ansprüche 1 bis 14, beschaffen zur Aufnahme wenigstens eines chirurgischen Manipulators, wenn das distale Ende des Tubus offen ist.

16. Tubus nach Anspruch 15, beschaffen zur Befestigung des chirurgischen Manipulators im Inneren des Tubus.

17. Tubus nach einem der Ansprüche 1 bis 16, weiterhin umfassend einen flüssigkeitsdichten Kanal, der konzentrisch im Tubus (10) angeordnet getragen wird, wobei der Kanal an seinem proximalen Ende verschlossen und an seinem distalen Ende offen ist und wobei der Umfangsrand des distalen Endes des Kanals in dichtem Eingriff mit einer am distalen Ende (14) des Tubus (10) ausgebildeten Pforte steht, wodurch eine Verbindungseinrichtung zwischen dem Inneren des Kanals und dem Äußeren des Tubus geschaffen wird.

18. Tubus nach Anspruch 17, wobei der Eingriff zwischen dem Kanal und dem Tubus ungefähr 5 bis 30 cm Wassersäule widerstehen kann.

19. Tubus nach Anspruch 17 oder 18, wobei der Kanal zur Aufnahme eines Teleskop beschaffen ist.

20. Tubus nach einem der Ansprüche 17 bis 19, wobei der Kanal zur Aufnahme wenigstens einer Lichtquelle beschaffen ist.

21. Tubus nach einem der Ansprüche 17 bis 20, weiterhin umfassend eine zweite Ventileinrichtung, die lösbar mit der Längswandung des Tubus in Eingriff ist.

## Revendications

1. Tube transvaginal (10) adapté pour être inséré dans la voie vaginale d'une patiente, et destiné à être utilisé au cours d'une hystérectomie coelioscopique ou autre opération de chirurgie coelioscopique effectuée sur la patiente utilisant la diathermie à électrocoagulation, le tube étant réalisé en une matière plastique substantiellement rigide mais flexible et ayant une extrémité distale (14) et une extrémité proximale (12), l'extrémité proximale étant coupée dans un plan (20, 22) non normal à son axe tubulaire grâce à quoi l'extrémité proximale forme un angle et est dimensionnée de manière à circonscrire la jonction cervico-vaginale (24) de la patiente afin de faciliter la séparation du vagin par diathermie à électrocoagulation, le tube comprenant en outre un moyen d'obturation (16) capable de former une obturation à l'extrémité distale (14) du tube pendant l'opération chirurgicale, le tube étant capable de maintenir le pneumopéritoine lorsqu'il est inséré dans la voie vaginale d'une patiente avec l'obturation formée à l'extrémité distale du tube.

2. Tube selon la revendication 1, dans lequel ladite obturation est sélectivement libérable.

3. Tube selon la revendication 1 ou 2, dans lequel l'extrémité proximale (12) du tube est biseautée.

4. Tube selon l'une quelconque des revendication 1 à 3, dans lequel l'extrémité proximale (12) a un bord lisse.

5. Tube selon l'une quelconque des revendication 1 à 4, dans lequel le tube (10) est transparent.

6. Tube selon l'une quelconque des revendication 1 à 4, dans lequel le tube (10) est opaque et comporte une ou plusieurs porte(s) transparente(s) sur sa longueur.

7. Tube selon l'une quelconque des revendication 1 à 6, dans lequel le moyen d'obturation (16) comprend un bouchon ou une capsule.

8. Tube selon la revendication 7, dans lequel le bouchon ou capsule est capable de former une obturation à l'extrémité distale du tube jusqu'à environ 5 à 30 cm de pression d'eau.

9. Tube selon la revendication 7, dans lequel le bouchon ou capsule est capable de former une obturation à l'extrémité distale du tube jusqu'à environ 15 cm de pression d'eau.

10. Tube selon l'une quelconque des revendication 1 à 6, dans lequel le moyen d'obturation (16) comprend un moyen formant soupape.

11. Tube selon la revendication 10, dans lequel le moyen formant soupape est en prise amovible avec l'extrémité distale du tube.

12. Tube selon la revendication 10 ou 11, dans lequel le moyen formant soupape est capable de former une obturation à l'extrémité distale du tube jusqu'à environ 5 à 30 cm de pression d'eau.

13. Tube selon la revendication 10 ou 11, dans lequel le moyen formant soupape est capable de former une obturation à l'extrémité distale du tube jusqu'à environ 15 cm de pression d'eau.

14. Tube selon l'une quelconque des revendication 1 à 13, adapté pour recevoir au moins un tube creux de diamètre inférieur au tube (10) quand l'extrémité distale du tube est ouverte.

15. Tube selon l'une quelconque des revendication 1 à 14, adapté pour recevoir au moins un manipulateur chirurgical quand l'extrémité distale du tube est ouverte.

16. Tube selon la revendication 15, adapté pour que le manipulateur chirurgical puisse être fixé à l'intérieur du tube.

17. Tube selon l'une quelconque des revendication 1 à 16, comprenant en outre un canal étanche aux fluides supporté de manière concentrique à l'intérieur du tube (10), le canal étant obturé en son extrémité proximale et ouvert en son extrémité distale et dans lequel le rebord périphérique de l'extrémité distale du canal est en prise étanche avec une porte formée dans l'extrémité distale (14) du tube (10), fournissant ainsi un moyen de communication entre l'intérieur du canal et l'extérieur du tube.

18. Tube selon la revendication 17, dans lequel la prise entre le canal et le tube est capable de supporter à peu près de 5 à 30 cm de pression d'eau.

19. Tube selon la revendication 17 ou 18, dans lequel le canal est adapté pour recevoir un télescope.

20. Tube selon l'une quelconque des revendication 17 à 19, dans lequel le canal est adapté pour recevoir au moins une source lumineuse.

21. Tube selon l'une quelconque des revendication 17 à 20, comprenant en outre un deuxième moyen formant soupape, mis en prise de manière amovible avec la paroi longitudinale du tube.
